(19) **Europäisches Patentamt / European Patent Office / Office européen des brevets**

(11) **EP 1 719 449 B1**

(12) **EUROPEAN PATENT SPECIFICATION**

(45) Date of publication and mention
of the grant of the patent:
**22.12.2010 Bulletin 2010/51**

(51) Int Cl.:
***A61B 5/00*** (2006.01)

(21) Application number: **06012571.3**

(22) Date of filing: **24.03.2000**

(54) **Improved pulse oximeter probe-off detector**

Verbesserter Detektor für Pulsoximetersondenablösung

Modèle amélioré de détecteur de décrochage de la sonde d'un spygmo-oxymètre

(84) Designated Contracting States:
**DE FR GB**

(30) Priority: **25.03.1999 US 126148 P**

(43) Date of publication of application:
**08.11.2006 Bulletin 2006/45**

(60) Divisional application:
**10181436.6**

(62) Document number(s) of the earlier application(s) in
accordance with Art. 76 EPC:
**00916663.8 / 1 171 025**

(73) Proprietor: **Masimo Corporation
Irvine, CA 92618 (US)**

(72) Inventors:
• **Diab, Mohamed K.
Mission Viejo, CA 92692 (US)**
• **Ali, Ammar Al
Trustin, CA 92782 (US)**

(74) Representative: **Vossius & Partner
Siebertstrasse 4
81675 München (DE)**

(56) References cited:
**US-A- 4 295 475     US-A- 4 399 824
US-A- 4 603 700     US-A- 5 368 041
US-A- 5 846 190**

**Description**

Background of the Invention

Oximetry is the measurement of the oxygen status of blood. Early detection of low blood oxygen is critical in the medical field, for example in critical care and surgical applications, because an insufficient supply of oxygen can result in brain damage and death in a matter of minutes. Pulse oximetry is a widely accepted noninvasive procedure for measuring the oxygen saturation level of arterial blood, an indicator of oxygen supply. A pulse oximetry system consists of a sensor attached to a patient, a monitor, and a cable connecting the sensor and monitor. Conventionally, a pulse oximetry sensor has both red and infrared (IR) light-emitting diode (LED) emitters and a photodiode detector. The sensor is typically attached to a patient's finger or toe, or a very young patient's patient's foot. For a finger, the sensor is configured so that the emitters project light through the fingernail and into the blood vessels and capillaries underneath. The photodiode is positioned at the fingertip opposite the fingernail so as to detect the LED transmitted light as it emerges from the finger tissues.

The pulse oximetry monitor (pulse oximeter) determines oxygen saturation by computing the differential absorption by arterial blood of the two wavelengths emitted by the sensor. The pulse oximeter alternately activates the sensor LED emitters and reads the resulting current generated by the photodiode detector. This current is proportional to the intensity of the detected light. The pulse oximeter calculates a ratio of detected red and infrared intensities, and an arterial oxygen saturation value is empirically determined based on the ratio obtained. The pulse oximeter contains circuitry for controlling the sensor, processing the sensor signals and displaying the patient's oxygen saturation and pulse rate. A pulse oximeter is described in U.S. Patent 5,632,272 assigned to the assignee of the present invention.

US5846190 refers to a pulse oximeter with several processing channels for measurement signals which are measured during the switched-on phase of transmission diodes and which are used to determine the oxygen saturation (SpO2) of a patient. An additional measurement channel processes an ambient light signal which was measured while the transmission diodes were switched off or their intensity modified, in the same way and thus provides a measure of the spectral composition of the ambient light interference. A noise-signal ratio is derived from the ambient light signal and a measurement signal, which represents a measure of the signal quality and which can be compared with threshold values, where an alarm is triggered if the value falls below the threshold values.

Summary of the Invention

To compute peripheral arterial oxygen saturation, denoted $Sp_2O_2$, pulse oximetry relies on the differential light absorption of oxygenated hemoglobin, $HbO_2$, and deoxygenated hemoglobin, Hb, to compute their respective concentrations in the arterial blood. This differential absorption is measured at the red and infrared wavelengths of the sensor. In addition, pulse oximetry relies on the pulsatile nature of arterial blood to differentiate hemoglobin absorption from absorption of other constituents in the surrounding tissues. Light absorption between systole and diastole varies due to the blood volume change from the inflow and outflow of arterial blood at a peripheral tissue site. This tissue site might also comprise skin, muscle, bone, venous blood, fat, pigment, etc., each of which absorbs light. It is assumed that the background absorption due to these surrounding tissues is invariant and can be ignored. Accordingly, blood oxygen saturation measurements are based upon a ratio of the time-varying or AC portion of the detected red and infrared signals with respect to the time-invariant or DC portion. This AC/DC ratio normalizes the signals and accounts for variations in light pathlengths through the measured tissue.

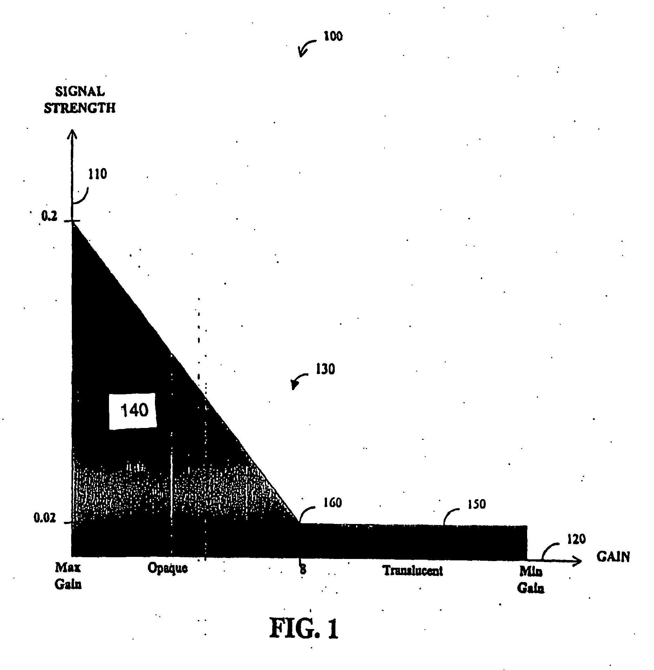

FIG. **1** illustrates the typical operating characteristics of a pulse oximeter. During a calibration phase, the pulse oximeter input gain is adjusted higher to accommodate opaque skin and lower to accommodate translucent skin at the sensor site. Variations in blood perfusion at the sensor site result in variations in input signal strength. The graph **100** shows acceptable input sensitivity as a function of gain. The y-axis **110** represents the signal strength (SS), which is the ratio of the peak-to-peak **AC** signal to the **DC** signal, expressed as a percentage. The x-axis **120** represents the gain, which is shown with decreasing values along the x-axis. The graph **100** has an unshaded region **130** representing the acceptable operating range of the pulse oximeter and a shaded region **140** representing conditions outside that operating range, which, when detected, will result in a pulse oximeter "probe off" alarm. The operating region **130** has a floor **150** at relatively low gains, representing the highest sensitivity to patients with low perfusion. Because input noise increases with gain, the operating region also has a corner point 160 below which input sensitivity is noise limited and falls off with increasing gain, i.e. increasing opacity.

A pulse oximeter with the operating characteristics shown in FIG. 1 may fail to detect a probe off condition. This problem occurs when the sensor becomes partially or completely dislodged from the patient, but continues to detect an AC signal within the operating region of the pulse oximeter. Probe off errors are serious because the pulse oximeter may display a normal saturation when, in fact, the probe is not properly attached to the patient, potentially leading to missed desaturation events.

Failure to detect a probe off condition is the result of the sensor detector receiving light directly from the emitters without transmission through the patient's tissue. The pulse oximeter is particularly vulnerable to probe off errors when operating at its highest sensitivity, where even small induced variations in light directly detected from the emitters have sufficient signal strength to be processed as a physiological signal. In a probe off condition, a detector **AC** signal can be induced by slight changes in the direct light path between the emitters and detector. For example, small amounts of patient motion, such as chest movement from breathing, can induce a probe off **AC** signal. As another example, "creep" in the sensor configuration, such as a folded sensor gradually returning to its original unfolded shape after becoming dislodged can also induce a probe off AC signal. Further restricting the operating region **130** shown in FIG. 1 can reduce probe off errors. Such restrictions, however, would also severely limit the ability of the pulse oximeter to make saturation measurements on patients with poor perfusion.

The invention is set out in independent claim 1. The present invention is a monitor-based improvement to detecting the probe off condition described above. Of-course, other methods of detecting the probe-off condition could be combined with the present improvement. In particular, an intelligent, rule-based processor uses signal quality measurements to limit the operating region of the pulse oximeter without significant negative impact on low perfusion performance. These signal-quality operating limits are superimposed on those of FIG. **1** to improve probe off detection. In this manner, the pulse oximeter can reject AC signals that have sufficient signal strength to fall within the operating region **130** of FIG. **1,** but that are unlikely to be a plethysmograph signal. One signal quality measurement that is used is pulse rate density, which is the percentage of time detected pulses satisfy a physiologically acceptable model. Another signal quality measurement is energy ratio, which is the percentage of signal energy that occurs at the pulse rate and its harmonics. The operating region of the pulse oximeter is then defined in terms of signal strength versus gain, signal strength versus PR density and energy ratio versus predefined energy ratio limits.

Brief Description of the Drawings

FIG. **1** is a graph illustrating minimum signal strength operating limits for a pulse oximeter;
FIGS. **2A** and **2B** are graphs illustrating additional minimum signal strength operating limits for a pulse oximeter, based on signal quality according to the present invention;

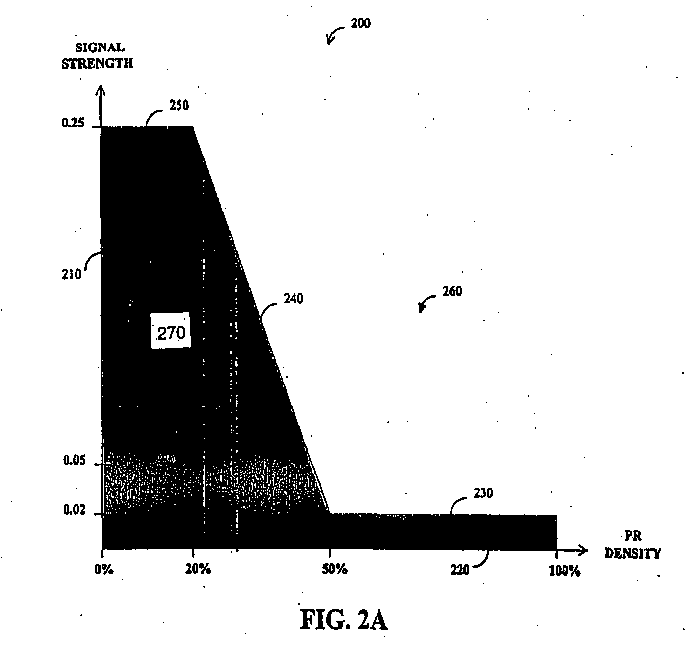

FIG. **2A** is a graph of signal quality operating limits for a pulse oximeter in normal input sensitivity mode;

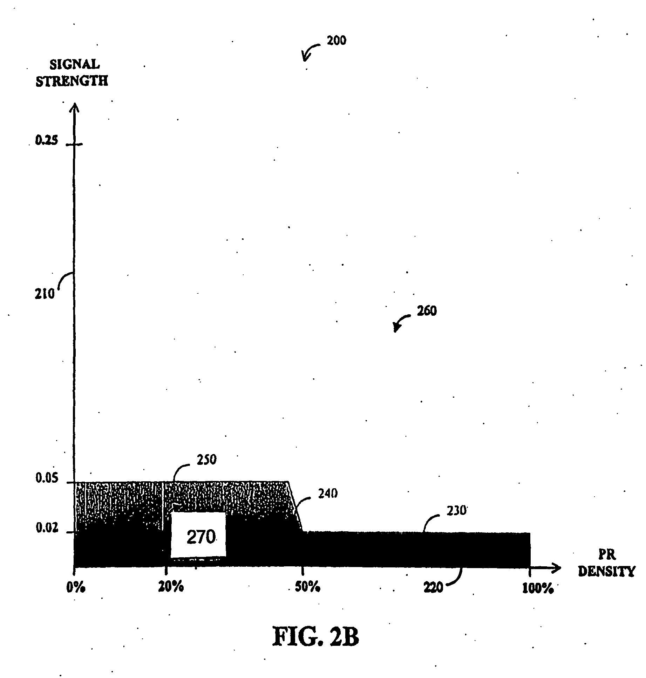

FIG. **2B** is a graph of signal quality operating limits for a pulse oximeter in high input sensitivity mode;

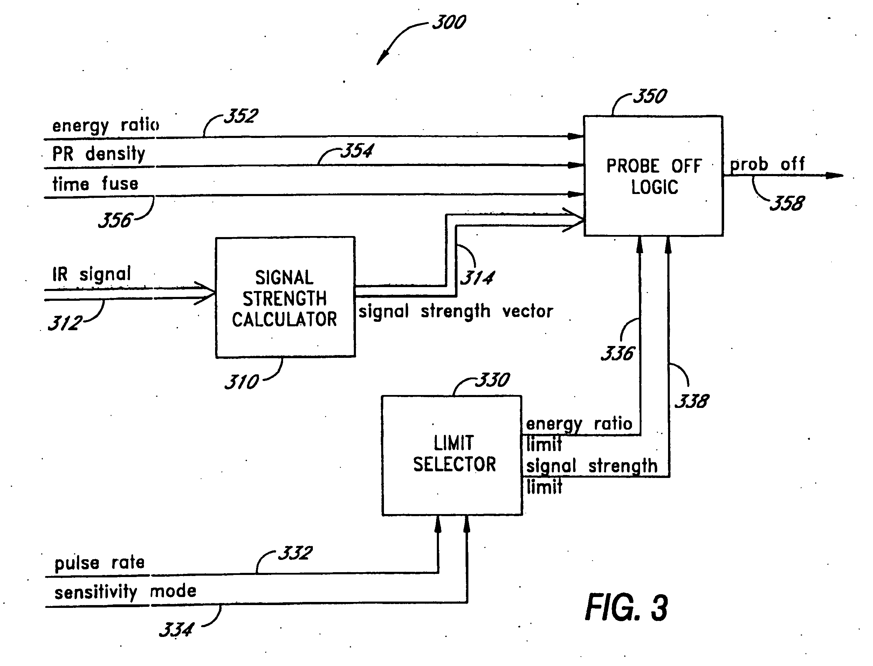

FIG. **3** is a top-level block diagram of a rule-based intelligent processor that provides the signal quality operating limits illustrated in FIGS. **2A-2B;**

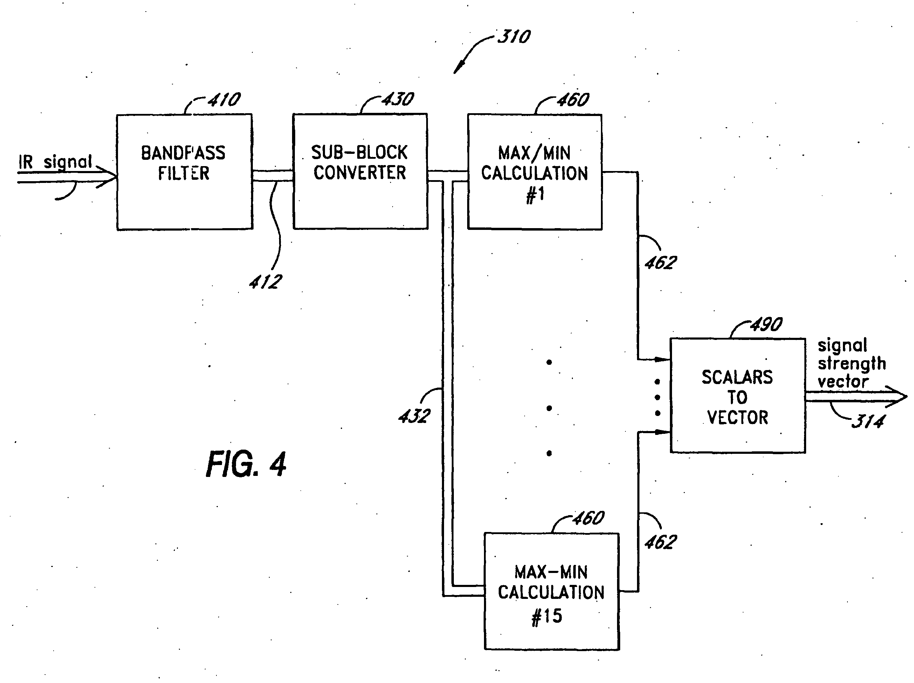

FIG. **4** is a detailed block diagram of the signal strength calculator portion of FIG. 3;

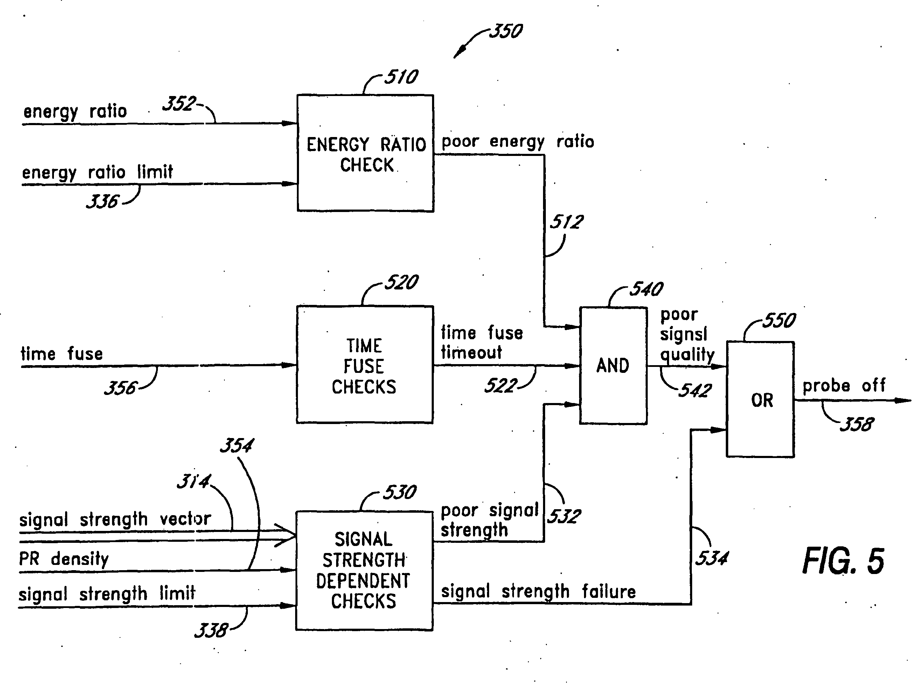

FIG. **5** is a detailed block diagram of the probe off logic portion of FIG. 3; and

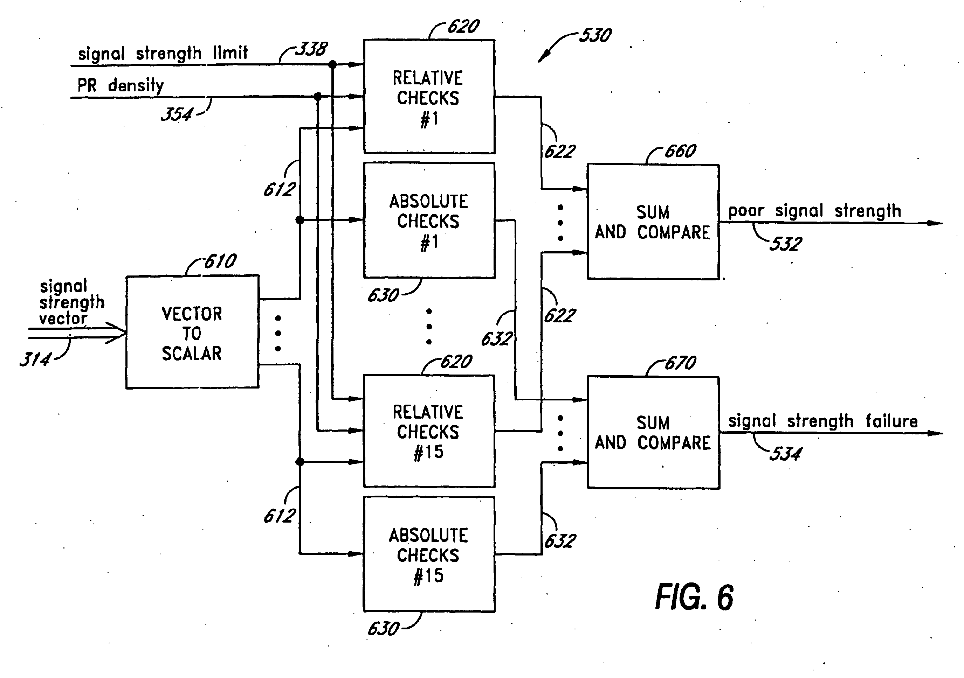

FIG. **6** is a detailed block diagram of the signal strength dependent checks portion of FIG. 5.

Detailed Description of the Preferred Embodiments

FIGS. **2A** and **2B** illustrate how the operating range of a pulse oximeter is modified based on pulse rate density according to one embodiment of the present invention. Calculation of PR density is disclosed in U.S. Provisional Patent Application No. 60/114,127 filed December 30, 1998, and in U.S. Patent Application No. 09/471,510, filed December 23, 1999, entitled "Plethysmograph Pulse Recognition Processor," which is assigned to the assignee of the current application. The processor described therein has a candidate pulse portion that determines a plurality of potential pulses within the input IR waveform. A physiological model portion of the processor then determines the physiologically acceptable ones of these potential pulses. The processor provides statistics regarding the acceptable pulses. One statistic is pulse density, which is the ratio of the period of acceptable pulses to the duration of a block or "snapshot" of the IR input waveform.

FIG. **2A** shows a graph **200** of signal strength on the y-axis **210** versus PR density on the x-axis **220** for normal sensitivity. The operating region **260** is shown unshaded, and the probe off region **270** is shown shaded. A signal strength floor 230 of .02, below which a probe off condition exists for all values of PR density, determines one portion of the operating region 260. That is, no matter how many of the detected plethysmograph pulses are deemed physiologically acceptable, if the signal strength is less than .02, then the pulse oximeter indicates a probe off condition. A signal strength ceiling 250 of .25, above which the pulse oximeter is in a valid operating region for all values of PR density, determines another portion of the operating region 260. That is, signal quality is ignored if signal strength is above .25. Between the signal strength ceiling 250 and floor 230, acceptable signal strength is dependent on PR density. The slope of the boundary 240 defining this relationship is:

EP 1 719 449 B1

$$slope = -(.25-.02)/(.5-.2) = -.23/.3 = -.7667 \qquad (1)$$

Thus, this boundary can be defined by the following equivalent equations:

$$SS = -.7667 \bullet PR\ density + .4033 \qquad (2)$$

$$PR\ density = -1.3043 \bullet SS + 0.5261 \qquad (3)$$

FIG. 2B shows a graph 200 of signal strength on the y-axis 210 versus PR density on the x-axis 220 for high sensitivity. This graph is equivalent to that of FIG. 2A except that the signal strength ceiling 250 is set at .05. Thus, signal quality indicated by PR density is ignored as long as the signal strength is above .05.

Another signal quality measure, energy ratio, is also imposed on the operating region as an absolute limit. Energy ratio is the percentage of IR signal energy occurring at the pulse rate and associated harmonics compared to total IR energy. The energy ratio is computed by transforming each block of the IR signal into the frequency domain as is well known in the art. The energy ratio is computed by identifying each peak in the resulting spectrum. In one embodiment, the peaks occurring at the pulse rate and its harmonics are identified and summed. This value is divided by the sum of the magnitudes of all peaks and output as the energy ratio. Note that energy ratio computed in this manner is not a true energy calculation because the calculations are based on the peak magnitudes and not the squared magnitudes of the IR signal. In this embodiment, the minimum energy ratio must be .6 if the pulse rate is greater than or equal to 30 and 5 otherwise. That is, 60% (or 50% for low pulse rates) of the signal must be at the pulse rate frequency or its harmonics or the pulse oximeter will indicate a probe off condition. A method for calculating the pulse rate used in this calculation is disclosed in U.S. Patent No. 6.002.952, filed April 14, 1997, entitled "Improved Signal Processing Apparatus and Method," which is assigned to the assignee of the current application.

FIG. 3 is a block diagram illustrating one embodiment of the improved probe-off detector 300 according to the present invention. The detector has a signal strength calculator 310, a limit selector 330 and probe-off logic 350. The signal strength calculator 310 has an IR signal 312 input. This signal is the detected sensor signal after demultiplexing, amplification, filtering and digitization. In a particular embodiment, the IR signal is input to the signal strength calculator 310 at a 625 Hz sample rate and in overlapping "snapshots" or blocks of 390 samples, each offset from the previous block by 25 samples. The signal strength calculator 310 creates a signal strength vector output 314 consisting of a set of signal strength scalars for each of these input blocks, as described with respect to FIG. 4 below.

The limit selector **330** has pulse rate **332** and sensitivity mode **334** inputs. When the sensitivity mode input **334** has a value of 1, it indicates that the pulse oximeter is in a normal sensitivity mode, corresponding to FIG. **2A.** A value of 0 indicates the pulse oximeter is in a high sensitivity mode, corresponding to FIG. **2B.** The pulse oximeter operator selects the sensitivity mode. The limit selector **330** also has energy ratio limit **336** and signal strength limit **338** outputs, which are input to the probe off logic **350** as absolute minimums of energy ratio and signal strength below which a probe off condition may be indicated **350**. The relationship between the pulse rate **332** and sensitivity mode **334** inputs and the energy ratio **336** and signal strength **338** outputs is specified below:

| INPUT STATE | SELECTED LIMIT |
|---|---|
| pulse rate $\geq$ 30 | minimum energy ratio = 0.6 |
| pulse rate < 30 | minimum energy ratio = 0.5 |
| sensitivity mode = 0 | minimum signal strength = 0.05 |
| sensitivity mode = 1 | minimum signal strength = 0.25 |

The probe off logic **350** has as inputs energy ratio **332, PR** density **334** and signal strength vector **314.** These inputs are compared to the energy ratio limit **336** and signal strength limit **338** outputs from the limit selector **330** to determine the operating region of the pulse oximeter. The probe off logic **350** also has a time fuse input **356.** The time fuse **356** is a counter that indicates the number of IR waveform blocks containing no acceptable pulses. Acceptable pulses are determined as described for the calculation of PR density **354,** above. The time fuse **356** input is 1 if there have been no acceptable pulses in a block since startup. The time fuse **356** is reset to 0 each time no acceptable pulses are detected for an input block. For each block where there are no acceptable pulses, the time fuse **356** is incremented by one. The time fuse enables the energy ratio limit and that portion of the signal strength limits above the floor **230** (FIGS. **2A-2B).** This reduces the probability of probe off alarms for transient events. In a particular embodiment, the time fuse **356** is compared to the constants -1 and 5. That is, the energy ratio and signal strength limits are enabled if there have been no acceptable pulses since startup or for more than the previous 5 IR signal blocks.

The probe off logic **350** has a Boolean probe off output **358** that is set to 1 when the probe off logic 350 detects the pulse oximeter is operating outside permissible limits. Otherwise, the probe off output **358** is 0. The probe off output can be used by the pulse oximeter to trigger a probe off alarm and error message to alert medical personnel to inspect and reattach the sensor or take other appropriate action. The probe off logic **350** is described in more detail below with respect to FIG. **5.**

FIG. **4** shows further details of the signal strength calculator **310** (FIG. **3**). Each **390** sample block of the IR signal **312** is initially filtered **410** remove any trends in the IR signal **312** that could cause an error in the signal strength calculations. In a particular embodiment, the filter **410** is a bandpass FIR filter with cutoff frequencies of **50** Hz and 550 Hz and a 151 tap Kaiser window having a shape parameter of 3.906. As a result, 150 samples are lost from each **390** sample input block. Thus, the filtered IR output **412** consists of **240** sample blocks.

Each **240** sample block of the filtered IR output **412** is converted **430** into multiple overlapping sub-blocks. In a particular embodiment, the sub-blocks each consist of 100 samples, and each sub-block is offset by 10 samples from the previous sub-block. Thus, the sub-block converter **430** creates 15 sub-block outputs **432** for each **240** sample filtered IR block **412.** For each sub-block, a max-min calculation **460** is performed. That is, the minimum sample magnitude in a particular sub-block is subtracted from the maximum sample magnitude in that sub-block. Each max-min output **462** is a single scalar representing the signal strength of a particular sub-block. A scalar-to-vector conversion **490** combines the max-min outputs **462** into a vector output **314** containing multiple signal strength values representing the signal strength of a particular block of the IR signal **312.**

FIG. **5** provides further detail of the probe off logic **350** (FIG. **3).** The probe off logic **350** has three functional checks that each provide a Boolean output. An energy ratio check **510** compares the energy ratio **352** against the energy ratio limit **336** provided by the limit selector **330** (FIG. **3),** specified in the table above. The energy ratio check **510** sets the "poor energy ratio" output **512** if the energy ratio **352** is below the energy ratio limit 336.

A time fuse check **520** determines if the time fuse **356** indicates no acceptable pulses have occurred in the IR signal **312** (FIG. **3)** for a sufficiently long time period. If so, a timeout output **522** is set. In a particular embodiment, the time fuse check **520** consists of comparators that determine if the time fuse **356** is -1 or greater than 5, indicating no acceptable pulses since startup or for a longer period than the past 5 blocks of IR signal **312.**

The signal strength dependent checks **530** determine if the pulse oximeter is within the operating limits described above with respect to FIGS. **2A** and **2B.** If the signal strength, as determined by the signal strength vector **314,** is below the floor **230** (**FIGS. 2A-B**), then the signal strength failure output **534** is set. If the signal strength is above the floor **230** (**FIGS. 2A-B**) but otherwise outside the operating region, i.e. within the shaded region 270 (FIGS. 2A-B) above the floor **230** (FIGS. **2A-2B),** then the "poor signal strength" output **532** is set.

A logical AND function **540** sets a "poor signal quality" output **542** if the poor energy ratio **512,** poor signal strength **532** and timeout **522** outputs are set. A logical OR function **550** sets the probe off output **358** if the poor signal quality **542** or the signal strength failure **534** outputs are set.

FIG. **6** shows a particular embodiment of the signal strength dependent checks **530** (FIG. **5).** The signal strength vector **314** is converted **610** into the 15 individual signal strength scalars **612.** Relative checks **620** and absolute checks **630** are performed on each of the 15 scalars **612.** Each relative check **620** determines if signal strength is within the signal strength limit **338** relative to PR density **354.** That is, each relative check output **622** is set according to the following, see Eq. 3 above:

| INPUT STATE | RESULT |
|---|---|
| SS $\geq$ SS limit | output =0 |
| PR density> -1.3043•SS+0.5261 | output = 0 |
| (SS< SS limit) AND PR density< -1.3043•SS+0.5261 | output =1 |

Each absolute check **630** determines if the signal strength is above the absolute minimum floor 230 (FIGS. **2A-2B**). That is, each absolute check output **632** is set according to the following:

| INPUT STATE | RESULT |
|---|---|
| SS $\geq$ 0.02 | output = 0 |
| SS < 0.02 | output = 1 |

The **15** relative check outputs **622** are processed by a sum and compare **660,** which performs an arithmetic sum of these outputs **622**. If the sum is equal or greater than 5, the poor signal strength output **532** is set. That is, poor signal strength is indicated if at least 1/3 of the scalars in the signal strength vector **314** fail their relative checks **620**. Likewise, the 15 absolute check outputs **632** are processed by a sum and compare **670,** which performs an arithmetic sum of these outputs **632**. If the sum is equal or greater than 5, the signal strength failure output **534** is set. That is, a signal strength failure is indicated if at least 1/3 of the scalars in the signal strength vector **314** fail the absolute checks **630**.

This improvement to detecting pulse oximetry probe off conditions has been disclosed in detail in connection with various embodiments of the present invention. These embodiments are disclosed by way of examples only and are not to limit the scope of the present invention, which is defined by the claims that follow. One of ordinary skill in the art will appreciate many variations and modifications within the scope of this invention.

**Claims**

1. A processor (300) for determining when an optical pulse oximetry sensor may not be properly positioned with respect to a measurement site, the processor comprising:

   a signal strength calculator (310) which processes a sensor signal input expected to be representative of at least one parameter measured by the optical pulse oximetry sensor, to produce a signal strength output representative of a strength of the sensor signal input, wherein the sensor signal input is provided from the optical pulse oximetry sensor; and
   a probe off logic module adapted to indicate that the sensor signal input may not represent the parameter, based on a comparison of the signal strength output and a signal quality input of said sensor signal input, wherein an acceptable operating region for the sensor involves signal strength output values dependent on the signal quality input and signal strength output values independent from said signal quality input.

2. The processor of Claim 1, wherein the probe off logic module (350) comprises a comparator.

3. The processor of Claim 1, wherein the signal strength output, involves a ratio of a first value of a substantially alternating part of the sensor signal input to a second value of a substantially non-alternating part of the sensor signal input.

4. The processor of Claim 3, further comprising a signal strength ceiling (250) above which a probe-off condition does not exist for all values of the sensor quality input.

5. The processor of Claim 3, wherein the ceiling (250) is 0.25.

6. The processor of Claim 3, wherein the ceiling (250) is 0.05.

7. The processor of Claim 3, wherein the probe off logic module (350) is adapted to indicate that the sensor signal input may not represent the parameter when the signal strength output is below the ceiling (250) and the signal quality and above a signal strength floor (230) input falls within a probe-off region.

8. The processor of Claim 7, wherein the probe-off region comprises a region defined by a relationship between the signal strength output and the signal quality input.

9. The processor of Claim 7, wherein the signal quality input comprises a comparison of the sensor signal input with one or more physiological signal models.

10. The processor of Claim 7, further comprising a timeout input indicative of non-acceptable pulses in at least one predetermined block of the sensor signal input

11. The processor of Claim 7, wherein the probe off logic module indicates that a probe-off condition exists when a timeout output indicates absence of acceptable pulses in at least one predetermined block of the sensor signal input, when the signal strength output is below the ceiling (250) and above the floor (230), and the signal quality of the sensor signal input falls within the probe-off region.

12. The processor of Claim 7, further comprising an energy ratio representative of whether a measurement of energy in the sensor signal input is above a limit value.

13. The processor of Claim 12, wherein the probe off logic module (350) is adapted to indicate that a probe-off condition may exist when the energy ratio is below the limit value, when the signal strength output is below the ceiling (250) and above the floor, and when the signal quality input falls within the probe-off region.

14. The processor of Claim 12, wherein the probe off logic module is adapted to indicate that a probe-off condition may exist when the energy ratio is below the limit value, a timeout output indicates absence of acceptable pulses in at least one predetermined block of the sensor signal input, when the signal strength output is below the ceiling (250) and above the floor (230), and when the signal quality of the sensor signal input falls within the probe-off region.

15. The processor of Claim 1, further comprising a signal strength floor below which a probe-off condition exists for all values of the signal quality input.

16. The processor of Claim 3, wherein the ceiling (250) is dependent upon a sensitivity mode of the sensor.

17. The processor of Claim 16, wherein the sensitivity mode is selectable by an operator.

**Patentansprüche**

1. Prozessor (300) zur Bestimmung, wann ein optischer Pulsoximetriesensor im Hinblick auf eine Meßstelle möglicherweise nicht richtig positioniert ist, wobei der Prozessor aufweist:

   einen Signalstärkerechner (310), der eine Sensorsignaleingabe verarbeitet, die erwartungsgemäß mindestens einen Parameter darstellt, der durch den optischen Pulsoximetriesensor gemessen wird, um eine Signalstärkeausgabe als Darstellung einer Stärke der Sensorsignaleingabe zu erzeugen, wobei die Sensorsignaleingabe vom optischen Pulsoximetriesensor bereitgestellt wird; und
   ein Sondenablösungs-Logikmodul (350), das geeignet ist, auf der Grundlage eines Vergleichs der Signalstärkeausgabe und einer Signalqualitätseingabe der Sensorsignaleingabe anzuzeigen, daß die Sensorsignaleingabe möglicherweise nicht den Parameter darstellt, wobei ein akzeptabler Betriebsbereich für den Sensor von der Signalqualitätseingabe abhängige Signalstärkeausgabewerte und von der Signalqualitätseingabe unabhängige Signalstärkeausgabewerte beinhaltet.

2. Prozessor nach Anspruch 1, wobei das Sondenablösungs-Logikmodul (350) einen Vergleicher aufweist.

3. Prozessor nach Anspruch 1, wobei die Signalstärkeausgabe ein Verhältnis eines ersten Werts eines im wesentlichen alternierenden Teils der Sensorsignaleingabe zu einem zweiten Wert eines im wesentlichen nicht alternierenden Teils der Sensorsignaleingabe beinhaltet.

4. Prozessor nach Anspruch 3, ferner mit einer Signalstärke-Höchstgrenze (250), über der kein Sondenablösungszustand für alle Werte der Sensorqualitätseingabe vorliegt.

5. Prozessor nach Anspruch 3, wobei die Höchstgrenze (250) 0,25 beträgt.

6. Prozessor nach Anspruch 3, wobei die Höchstgrenze (250) 0,05 beträgt.

7. Prozessor nach Anspruch 3, wobei das Sondenablösungs-Logikmodul (350) geeignet ist anzuzeigen, daß die Sensorsignaleingabe möglicherweise nicht den Parameter darstellt, wenn die Signalstärkeausgabe unter der Höchstgrenze (250) und über einer Signalstärke-Mindestgrenze (230) liegt und die Signalqualitätseingabe in einen Sondenablösungsbereich fällt.

8. Prozessor nach Anspruch 7, wobei der Sondenablösungsbereich einen Bereich aufweist, der durch eine Beziehung zwischen der Signalstärkeausgabe und der Signalqualitätseingabe festgelegt ist.

9. Prozessor nach Anspruch 7, wobei die Signalqualitätseingabe einen Vergleich der Sensorsignaleingabe mit einem oder mehreren physiologischen Signalmodellen aufweist.

10. Prozessor nach Anspruch 7, ferner mit einer Zeitablaufeingabe als Anzeige für inakzeptable Impulse in mindestens einem vorbestimmten Block der Sensorsignaleingabe.

11. Prozessor nach Anspruch 7, wobei das Sondenablösungs-Logikmodul anzeigt, daß ein Sondenablösungszustand vorliegt, wenn eine Zeitablaufausgabe das Fehlen von akzeptablen Impulsen in mindestens einem vorbestimmten Block der Sensorsignaleingabe anzeigt, wenn die Signalstärkeausgabe unter der Höchstgrenze (250) und über der Mindestgrenze (230) liegt und die Signalqualität der Sensorsignaleingabe in den Sondenablösungsbereich fällt.

12. Prozessor nach Anspruch 7, ferner mit einem Energieverhältnis als Darstellung dafür, ob eine Energiemessung in der Sensorsignaleingabe über einem Grenzwert liegt.

13. Prozessor nach Anspruch 12, wobei das Sondenablösungs-Logikmodul (350) geeignet ist anzuzeigen, daß ein Sondenablösungszustand möglicherweise vorliegt, wenn das Energieverhältnis unter dem Grenzwert liegt, wenn die Signalstärkeausgabe unter der Höchstgrenze (250) und über der Mindestgrenze liegt und wenn die Signalqualitätseingabe in den Sondenablösungsbereich fällt.

14. Prozessor nach Anspruch 12, wobei das Sondenablösungs-Logikmodul geeignet ist anzuzeigen, daß ein Sondenablösungszustand möglicherweise vorliegt, wenn das Energieverhältnis unter dem Grenzwert liegt, eine Zeitablaufausgabe das Fehlen akzeptabler Impulse in mindestens einem vorbestimmten Block der Sensorsignaleingabe anzeigt, wenn die Signalstärkeausgabe unter der Höchstgrenze (250) und über der Mindestgrenze (230) liegt und wenn die Signalqualität der Sensorsignaleingabe in den Sondenablösungsbereich fällt.

15. Prozessor nach Anspruch 1, ferner mit einer Signalstärke-Mindestgrenze, unter der ein Sondenablösungszustand für alle Werte der Signalqualitätseingabe vorliegt.

16. Prozessor nach Anspruch 3, wobei die Höchstgrenze (250) von einem Empfindlichkeitsmodus des Sensors abhängt.

17. Prozessor nach Anspruch 16, wobei der Empfindlichkeitsmodus von einem Bediener auswählbar ist.

**Revendications**

1. Processeur (300) pour déterminer quand un détecteur d'oxymétrie à impulsion optique peut ne pas être correctement positionné relativement à un site de mesure, le processeur comprenant :

   ■ un calculateur de résistance de signal (310) qui traite une entrée de signal de capteur supposée représentative d'au moins un paramètre mesuré par le capteur d'oxymétrie à impulsion optique, afin de produire une sortie de résistance de signal représentative d'une résistance de l'entrée de signal de capteur, dans lequel l'entrée de signal de capteur est fournie par le capteur d'oxymétrie à impulsion optique ; et
   ■ un module logique de décrochage de sonde (350), adapté pour indiquer que l'entrée du signal de capteur peut ne pas représenter le paramètre, sur la base d'une comparaison de la sortie de la résistance de signal et d'une entrée de qualité de signal de ladite entrée de signal de capteur, dans lequel une zone opérationnelle acceptable pour le capteur implique des valeurs de sortie de la résistance de signal dépendantes de l'entrée de qualité de signal et des valeurs de sortie de la résistance de signal indépendantes de ladite entrée de qualité de signal.

2. Processeur selon la revendication 1, dans lequel le module logique de décrochage de sonde (350) comprend un comparateur.

3. Processeur selon la revendication 1, dans lequel la sortie de la résistance de signal implique un rapport entre une première valeur d'une partie sensiblement alternative de l'entrée de signal de capteur et une seconde valeur d'une partie sensiblement non alternative de l'entrée de signal de capteur.

4. Processeur selon la revendication 3, comprenant en outre un plafond de résistance de signal (250) au dessus duquel une condition de décrochage de sonde n'existe pas pour toutes les valeurs de l'entrée de qualité de capteur.

5. Processeur selon la revendication 3, dans lequel le plafond (250) est de 0,25.

6. Processeur selon la revendication 3, dans lequel le plafond (250) est de 0,05.

7. Processeur selon la revendication 3, dans lequel le module logique de décrochage de sonde (350) est adapté pour indiquer que l'entrée du signal de capteur peut ne pas représenter le paramètre quand la sortie de la résistance de signal est inférieure au plafond (250) et supérieure à une limite inférieure de résistance de signal (230) et l'entrée de qualité de signal tombe dans une zone de décrochage de sonde.

8. Processeur selon la revendication 7, dans lequel la zone de décrochage de sonde comprend une zone définie par une relation entre la sortie de résistance de signal et l'entrée de qualité de signal.

9. Processeur selon la revendication 7, dans lequel l'entrée de qualité de signal comprend une comparaison de l'entrée de signal de capteur avec un ou plusieurs modèles de signal physiologiques.

10. Processeur selon la revendication 7, comprenant en outre une entrée de temporisation indicative d'impulsions non-acceptables dans au moins un bloc prédéterminé de l'entrée du signal de capteur.

11. Processeur selon la revendication 7, dans lequel le module logique de décrochage de sonde indique qu'une condition de décrochage de sonde existe quand une sortie de temporisation indique l'absence d'impulsions acceptables dans au moins un bloc prédéterminé de l'entrée de signal de capteur, quand la sortie de résistance de signal est inférieure au plafond (250) et supérieure à la limite inférieure (230), et la qualité de signal de l'entrée de signal de capteur tombe dans la zone de décrochage de sonde.

12. Processeur selon la revendication 7, comprenant en outre un rapport d'énergie représentatif du fait si une mesure de l'énergie dans l'entrée du signal de capteur est supérieure à une valeur limite.

13. Processeur selon la revendication 12, dans lequel le module logique de décrochage de sonde (350) est adapté pour indiquer qu'une condition de décrochage de sonde peut exister quand le rapport énergétique est inférieur à la valeur limite, quand la sortie de résistance du signal est inférieure au plafond (250) et supérieure à la limite inférieure, et quand l'entrée de qualité de signal tombe dans la zone de décrochage de sonde.

14. Processeur selon la revendication 12, dans lequel le module logique de décrochage de sonde est adapté pour indiquer qu'une condition de décrochage de sonde peut exister quand le rapport énergétique est inférieur à la valeur limite, une sortie de temporisation indique l'absence d'impulsions acceptables dans au moins un bloc prédéterminé de l'entrée de signal de capteur, quand la sortie de résistance de signal est inférieure au plafond (250) et supérieure à la limite inférieure (230) et quand la qualité de signal de l'entrée de signal de capteur tombe dans la zone de décrochage de sonde.

15. Processeur selon la revendication 1, comprenant en outre une limite inférieure de résistance de signal en deçà de laquelle une condition de décrochage de sonde existe pour toutes les valeurs de l'entrée de qualité de signal.

16. Processeur selon la revendication 3, dans lequel le plafond (250) dépend d'un mode de sensibilité du capteur.

17. Processeur selon la revendication 16, dans lequel le mode sensibilité peut être sélectionné par un opérateur.

**FIG. 1**

FIG. 2A

FIG. 2B

FIG. 3

FIG. 4

IR signal → BANDFASS FILTER (410) →412→ SUB-BLOCK CONVERTER (430) → MAX/MIN CALCULATION #1 (460) →462→ SCALARS TO VECTOR (490) → signal strength vector (314)

MAX-MIN CALCULATION #15 (460) →462→ SCALARS TO VECTOR

310, 432

FIG. 5

EP 1 719 449 B1

FIG. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5632272 A
- US 5846190 A
- US 11412798 P
- US 47151099 A
- US 6002952 A